(19) Europäisches Patentamt
European Patent Office
Office européen des brevets

(11) **EP 4 782 429 A1**

(12) **EUROPEAN PATENT APPLICATION**
published in accordance with Art. 153(4) EPC

(43) Date of publication:
**29.07.2026 Bulletin 2026/31**

(21) Application number: **24868378.1**

(22) Date of filing: **20.09.2024**

(51) International Patent Classification (IPC):
*C07C 57/15* (2006.01)   *B01J 20/22* (2006.01)
*B01J 20/30* (2006.01)   *C07C 63/16* (2006.01)
*C08K 5/56* (2006.01)   *C08L 101/00* (2006.01)
*C07F 3/06* (2006.01)   *C07F 5/06* (2006.01)

(52) Cooperative Patent Classification (CPC):
**B01J 20/22; B01J 20/30; C07C 57/15; C07C 63/16;
C08K 5/56; C08L 101/00;** C07F 3/06; C07F 5/06

(86) International application number:
**PCT/JP2024/033770**

(87) International publication number:
**WO 2025/063302 (27.03.2025 Gazette 2025/13)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **22.09.2023 JP 2023158002**

(71) Applicant: **Youzeo Co., Ltd.**
**Tokyo 113-0033 (JP)**

(72) Inventor: **YAMAZAKI, Yasuo**
**Tokyo 113-0033 (JP)**

(74) Representative: **Paustian & Partner**
**Patentanwälte mbB**
**Oberanger 32**
**80331 München (DE)**

Remarks:
A request for correction of the description has been filed pursuant to Rule 139 EPC. A decision on the request will be taken during the proceedings before the examining division (Guidelines for Examination in the EPO, A-V, 3).

(54) **ORGANIC METAL STRUCTURE, METHOD FOR PRODUCING SAME, GAS ADSORPTION MATERIAL USING SAME, AND METHOD FOR PRODUCING GAS ADSORPTION MATERIAL**

(57)    Provided is a production method for a metal-organic framework using a crystallizer including a reagent introduction zone, a crystallization zone, a circulation zone, and a recovery zone. In the reagent introduction zone, an aqueous solution containing a metal ion and an aqueous solution containing an organic ligand capable of coordinating to the metal ion are simultaneously and continuously added through different nozzles installed in a flow path, and are immediately mixed using a mixing mechanism. In the crystallization zone, the metal ion and the organic ligand are reacted to grow crystals of the metal-organic framework while simultaneously nucleating the crystals of the metal-organic framework. In the circulation zone, a slurry containing the crystals of the metal-organic framework is circulated to the reagent introduction step. In the recovery zone, a portion of the slurry is withdrawn continuously or at predetermined intervals.

Fig. 1

**EP 4 782 429 A1**

## Description

Technical Field

The present invention relates to a metal-organic framework and a production method for the same. The present invention also relates to a gas adsorption material using a metal-organic framework, and a production method for the same.

Background Art

A metal-organic framework (hereinafter also referred to as "MOF"), also called a porous coordination polymer (PCP), is a porous material having a highly ordered lattice structure formed from metal ions and organic ligands. Utilizing the highly ordered lattice structure of MOFs, MOFs are expected to be applied in, for example, gas storage materials such as hydrogen, carbon dioxide concentration and separation materials, heterogeneous catalysts, conductive materials, and magnetic materials.

An MOF can be synthesized, for example, by a solution method in which the metal ion and the organic ligand are self-assembled in a liquid (e.g., see Non-Patent Literature 1).

In this case, the solution is allowed to stand in a sealed vessel and is kept at a temperature higher than the evaporation temperature of a solvent, thereby causing the solution to volatilize and increase its internal pressure. Under high pressure, an MOF is synthesized by using the molecular volume of the MOF being smaller than the molecular volume of the solute as a driving force (referred to as a hydrothermal synthesis method in the case of a water solvent, and as a solvothermal method in the case of an organic solvent). Also, an MOF can be synthesized by continuously feeding, into a stirred tank, a solution of the metal ion dissolved in an organic solvent and water, and a solution of organic ligand dissolved in an organic solvent, and stirring the resulting solution. Further, an MOF can be synthesized by mixing the solutions in a tubular microreactor.

In either the hydrothermal synthesis method or the solvothermal method, in the case of batch reaction, the concentrations of the metal ion and the organic ligand ion in the solution at an initial stage of MOF synthesis are high, and the solute concentration inevitably decreases as the reaction proceeds. Since a maximum supersaturation degree is obtained at an initial stage of mixing the metal ion and the organic ligand ion, the crystal growth rate should reach the maximum at such a supersaturation degree. However, in reality, the supersaturation degree as the driving force is consumed by the nucleation rate, resulting in precipitation of a large number of crystals with small particle sizes. Further, as crystallization progresses, the solute concentration decreases and the supersaturation degree is lost, and ultimately, the crystals hardly grow. As a result, the production rate of the MOF per unit volume and per unit time becomes extremely low. This issue also applies to the case of continuous reaction as in a tubular microreactor in that although back-mixing of the solution occurs, the solute concentration in the reaction field changes from a high concentration to a low concentration.

In contrast, carrying out continuous stirred reaction in a stirred tank is an excellent method that maintains reaction conditions (e.g., solute concentration, temperature, or pH) constant when a steady state is reached. However, no method has been proposed for controlling the nucleation rate and the linear growth rate of MOF crystals within predetermined ranges. Crystallizers in which reactants are continuously fed into a stirred tank and products are continuously withdrawn are common. Particularly, in so-called MSMPR (Mixed Suspension Mixed Product Removal)-type crystallizers in which an entire stirred tank is maintained at a substantially uniform concentration, the MOF reaction occurs simultaneously with dripping, causing the reaction solution in the apparatus to rapidly change into fine MOF particles. Thus, the supersaturation degree of the reaction solution cannot be maintained at a high level. As a result, the resulting MOF crystals are crystals with a high proportion of fine particles and a low bulk density.

Note that when crystallizing ionic reactants, the pH is commonly an important parameter, and it has been reported that crystallization is carried out in a buffer solution in order to maintain a constant pH, which is also the case for MOFs. However, the number of ionic species in the reaction field increases, leading to a drawback in that the reaction field becomes a complex system.

An organic ligand refers to an organic material that has at least two carboxyl groups and contains two or more, typically four or more, carbon atoms, and is stable at room temperature and atmospheric pressure and at a relative humidity of 50%. Examples include fumaric acid, terephthalic acid, trimesic acid, triazole, and oxalic acid. To synthesize an MOF, it is necessary to ionize the organic material and metal salt and form a coordination bond between the metal and the carboxyl group of the organic material. To simultaneously dissolve these two types of ions, it is common to use an organic solvent such as DMF (dimethylformamide), DEF (diethylformamide), or NMP (N-methyl-2-pyrrolidone). However, such organic solvents are more expensive than water. Moreover, the MOF cannot be applied in fields such as pharmaceuticals or foods unless the organic solvent is completely washed and separated from the produced MOF.

As another method, Patent Literature 1 proposes a method for producing an MOF in a short time by applying centrifugal force and shear force to a mixture of a metal ion donor serving as a raw material, a multidentate ligand, and a solvent.

Citation List

Patent Literature

Patent Literature 1: U.S. Patent Application Publication No. 2022/0220129A1

Non-Patent Literature

Non-Patent Literature 1: "Basics of Materials Science," Vol. 7, "Basics of Porous Coordination Polymers (PCP)/Metal-Organic Frameworks (MOF)," Sigma-Aldrich Japan, September 2012, Internet <URL:https://www.sigmaaldrich.com/JP/ja/campaigns/materials-science-basic>

Summary of Invention

In the hydrothermal synthesis method and the solvothermal method, as the temperature increases, water or the solvent volatilizes and the pressure thereof increases. In the absence of crystals, the solute concentration is high but the pressure is low. Thus, the supersaturation degree gradually increases before nucleation occurs. When the supersaturation degree reaches a certain level, nucleation occurs all at once, and a large number of crystals with small particle sizes are obtained. Thereafter, the pressure further increases and the supersaturation degree also increases, but the solute concentration decreases and no solute remains available for crystal growth at that stage. This results in the formation of a large number of crystals with small particle sizes. With only MOFs formed from such crystals with small particle sizes, the bulk density is low, and the amount of MOF per unit volume of the apparatus decreases when the MOF is packed into the apparatus. As a result, the adsorption amount decreases.

To solve this problem, a method is known that increases the particle size by the so-called Ostwald ripening phenomenon, which is a phenomenon in which fine particles dissolve and coarse particles grow as a result of maintaining the supersaturation degree for a long time under high temperature and high self-pressure. However, this method requires a long time, and consequently has an industrial problem in that the production rate per unit time decreases drastically.

In the case of tubular microreactors as well, although the crystals and the reaction solution move together in the apparatus, the same phenomenon occurs as those observed in the batch-type hydrothermal synthesis method and solvothermal method. Due to the above-described phenomenon, an MOF with small particle sizes is formed, and the adsorption amount per unit volume decreases.

When MOFs are used for the aforementioned adsorption applications, it is important for achieving high performance in those applications that the crystallinity degree is high and the packing properties of the particles is high.

Accordingly, an object of the present invention is to provide an MOF and a production method for the same, as well as a gas adsorption material using an MOF and a production method for the same, all of which can eliminate the above-described drawbacks of the conventional techniques.

Although MOFs can be produced according to the various production methods described above, the MOFs produced by those methods have particles with small particle sizes and a low bulk density, resulting in insufficient packing properties. In addition, the pore structure of a porous material is a one-dimensional or multidimensional pore structure, and as the length of the pore structure increases, the adsorption/desorption rate tends to become faster due to the capillary effect. That is, the higher the crystallinity degree is, the faster the adsorption/desorption rate is. Therefore, the requirements of the crystal structure for crystals of porous materials such as MOFs are that the particle size distribution be broad such that smaller crystal particles enter spaces between larger crystal particles, thereby increasing the bulk density, and that the crystallinity degree be high such that the adsorption/desorption rate becomes fast. It is also self-evident that the fewer impurities contained in an MOF are, the more stably adsorption and desorption can be performed.

To satisfy such requirements, a method may be adopted that grows an MOF in which the particle size distribution is broad, i.e., crystal particles with large particle sizes are contained, while both crystal particles having large particle sizes and those having small particle sizes are present, and the crystallinity degree is high at the same time. The present inventor diligently examined the apparatus shape for producing such an MOF and succeeded in realizing this through repeated experimentation. Furthermore, the inventor found a method by which washing for removing impurities can be performed on a large scale with a small amount of solvent and in a short period of time. In addition, the inventor found an industrially useful gas adsorption material and a suitable production method of this gas adsorption material by granulating the obtained metal-organic framework.

A characteristic of the crystallization phenomenon of an MOF formed by a double decomposition reaction in an aqueous solution system is that it involves almost no heat of crystallization.

The double decomposition reaction referred to herein is a reaction in which, when the metal of the MOF is represented as M, the organic ligand as O, and their respective counterions as X and Y, a reaction $MX_l + O_mY_n \rightarrow MO_m + X_lY_n$ is carried out to obtain an MOF compound ($MO_m$) and a counterion compound ($X_lY_n$).

It is a surprising characteristic that, in the case of MOFs, this commonly used double decomposition reaction involves almost no heat of crystallization. In general, in crystallization phenomena, when a reactant undergoes a phase change from a solution to a crystal of a product and becomes solid, heat is generated equal to dissolution heat. One possibility is that the phase change of the counter ion in the double decomposition reaction includes the dissolution and precipitation thereof in the case of sodium sulfate, and involves an endothermic reaction at temperatures of 33°C or higher. However, the concentration of the MOF in the double decomposition reaction is in a range much more dilute than the solubility of sodium sulfate, and therefore no phase change occurs. Thus, this possibility is negated.

The driving force of the crystallization phenomenon is the supersaturation degree. Since the supersaturation degree mainly depends on temperature and concentration, the supersaturation degree at a crystal growth interface decreases when heat is generated by a crystallization phenomenon. However, in the crystallization of MOFs, almost no heat of crystallization is generated, and the supersaturation degree can therefore be accurately controlled if the concentration can be properly controlled.

Next, when the crystal growth rate of an MOF was actually measured in the present invention, the crystal growth rate was naturally proportional to the supersaturation degree. However, although coarse particles were obtained, the crystal growth rate was unexpectedly small. It was also found that increasing the supersaturation degree increased the crystal growth rate. This phenomenon is occasionally observed when the solubility of the product is low, where a nucleation phenomenon occurs before a growth phenomenon occurs, and the supersaturation degree is not consumed by the growth phenomenon. However, in the case of porous materials, the apparent linear growth rate often increases because water is contained in the pores, and thus such phenomenon does not necessarily occur. Nevertheless, the inventor has discovered for the first time that, in the case of MOFs, which are porous materials, the balance between the nucleation rate and the crystal growth rate is unexpectedly dominated by the nucleation rate, and a considerably large supersaturation degree is required to obtain a high crystal growth rate, as their unique phenomenon.

In the present invention, an apparatus configuration for obtaining coarse MOF crystals was newly devised by utilizing the newly discovered relationship between the linear growth rate and the nucleation rate of MOFs. That is, a reagent introduction zone and a crystallization zone are provided in a part of a crystallizer, and in the reagent introduction zone, a solution containing a metal ion and a solution containing an organic ligand are rapidly introduced and mixed to obtain a large supersaturation degree. Crystal particles contained in a slurry are then passed through and mixed with the solution having the large supersaturation degree to grow the crystals epitaxially on the surfaces of the crystal particles, thereby producing crystals with large crystallite sizes and large particle sizes.

On the other hand, the rotational speed of stirring for mixing in the reagent introduction zone and the nucleation rate correlate to some extent. That is, increasing the rotational speed of stirring to improve mixing tends to increase the nucleation rate and increase the number of MOF crystal particles having small particle sizes. The inventor has devised a method in which, subsequently, the crystals are grown or the generated fine particles are attached to the crystal surfaces in the crystallization zone in accordance with the growth rate of the MOF, thereby incorporating an unnecessarily large number of nuclei into the crystal surfaces and reducing the number of nuclei to produce coarse MOF crystals.

It is possible that, in addition to stirring blades in the reagent introduction zone, an impeller for transporting the reaction solution is attached on the same axis, that is, a so-called multistage impeller is used. This makes it possible to separately control stirring conditions and the transport rate in the reagent introduction zone. When a multistage impeller was tested in the present invention, no remarkable effect was observed.

By making an MOF into coarse crystals, the specific surface area of the crystals decreased, making it possible to reduce the amount of mother liquor adhering to the crystal surfaces. This facilitated washing of the crystals.

Furthermore, as a method for separating the MOF crystals from the mother liquor, separation using a large centrifugal force is conceivable. Although the mechanism of this phenomenon is not yet elucidated, the inventor newly found that improving the washability of the crystals using this method could reduce the required amount of washing liquid.

The present invention provides a metal-organic framework comprising:
a crystal containing an organic ligand and a metal ion,

wherein the organic ligand is a fumaric acid dianion or a terephthalic acid dianion,
the metal ion is an aluminum ion, a zinc ion, or an iron(III) ion, and
the metal-organic framework has a tap density of 0.3 g/mL or more and 0.9 g/mL or less.

The present invention also provides a gas adsorption material comprising a granulated body of a mixture including: the metal-organic framework according to claim 1 or 2 as a gas adsorption component; and a phenolic resin as a shape-retaining component.

The present invention also provides a production method for producing a metal-organic framework using a crystallizer including a reagent introduction zone, a crystallization zone, a circulation zone, and a recovery zone,

the method comprising:

in the reagent introduction zone, simultaneously and continuously adding a first aqueous solution containing a metal ion and a second aqueous solution containing an organic ligand capable of coordinating to the metal ion through different nozzles installed in a flow path, and immediately mixing the first aqueous solution and the second aqueous solution using a mixing mechanism;
in the crystallization zone, reacting the metal ion and the organic ligand to grow a crystal of a metal-organic framework and simultaneously nucleate the crystal of the metal-organic framework;
in the circulation zone, circulating a slurry containing the crystal of the metal-organic framework to a reagent introduction step; and
in the recovery zone, withdrawing a portion of the slurry continuously or at a predetermined interval.

The present invention further provides a continuous washing method for continuously washing the metal-organic framework produced by the method according to claim 5,

the continuous washing method comprising:
a washing step of washing the metal-organic framework with a washing liquid to wash and remove a counteranion for dissolving the metal ion constituting the metal-organic framework and a countercation for dissolving the organic ligand,
wherein the washing step includes a first washing step, a solid-liquid separation step, and a second washing step, and a liquid that has been subjected to the first washing step is separated into a solid component and a liquid component in the solid-liquid separation step, the solid component is further washed in the second washing step, and the liquid component is used as a part of a washing liquid in the first washing step.

The present invention further provides a production method for producing a gas adsorption material, comprising:

a step of mixing the metal-organic framework produced by the method according to claim 5 with a thermosetting phenolic resin to obtain a mixture;
a step of adding water to the mixture and performing tumbling granulation to obtain granulated materials;
a step of separating the granulated materials that have grown to a predetermined particle size, in accordance with particle sizes thereof; and
a step of again subjecting the granulated materials that do not reach the predetermined particle size to the granulating step, heating the granulated materials that have reached the predetermined particle size at a first temperature to remove the water, and further heating the granulated materials that have reached the predetermined particle size to a second temperature to cure the thermosetting phenolic resin.

Brief Description of Drawings

[Fig. 1] Fig. 1 is a schematic diagram showing an apparatus suitably used for implementing a production method of the present invention.
[Fig. 2] Fig. 2 is a schematic diagram showing an example of a reaction apparatus suitably used for implementing the production method of the present invention.
[Fig. 3] Fig. 3(a) and Fig. 3(b) are optical microscope photographs of MOF crystals of the present invention.
[Fig. 4] Fig. 4 is a graph showing the results of a nitrogen adsorption-desorption test of spherical granulated material of Example 4.
[Fig. 5] Fig. 5 is a graph showing the results of a carbon dioxide adsorption test of a metal-organic framework of Example 1 and a spherical granulated material of Example 4.
[Fig. 6] Fig. 6 is a schematic diagram showing a continuous washing apparatus suitably used for implementing the production method of the present invention.

Description of Embodiments

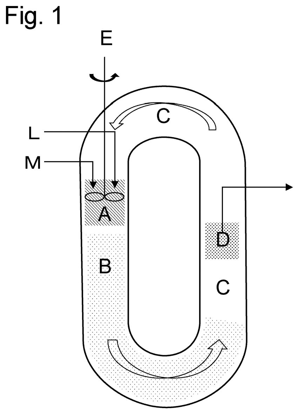

The present invention will be described below based on preferred embodiments thereof with reference to Fig. 1.
The present invention relates to a production method for producing a metal-organic framework, i.e., an MOF, formed from crystals containing an organic ligand and a metal ion. The present invention provides a continuous production method for an MOF. For this purpose, the production method suitably uses a crystallizer that includes a reagent introduction zone A, a crystallization zone B, a circulation zone C, and a recovery zone D. The production method using this crystallizer can be broadly divided into the following steps (1) to (4).

(1) Reagent introduction step

In this step, in the reagent introduction zone A, an aqueous solution M containing a metal ion and an aqueous solution L containing an organic ligand capable of coordinating to the metal ion are simultaneously and continuously added through different nozzles installed in a flow path and are immediately mixed using a mixing mechanism E.

(2) Crystallization step

In this step, in the crystallization zone B, the metal ion and the organic ligand are reacted to grow metal-organic framework crystals and simultaneously to generate crystal nuclei of the metal-organic framework.

(3) Crystal circulation step

In this step, in the circulation zone C, the slurry containing the metal-organic framework crystals is circulated to the reagent introduction step. That is, the metal-organic framework produced by the reaction is smoothly transferred to the reagent introduction step, and both the crystals and the solution are circulated.

(4) Crystal recovery step

In this step, in the recovery zone D, a portion of the slurry is withdrawn continuously or at predetermined intervals.
Note that additional steps may also be performed, as needed, before the step (1), between the steps (1) and (2), between the steps (2) and (3), between the steps (3) and (4), and/or after the step (4).
By combining the steps described herein, it becomes possible, despite the newly discovered phenomenon that the growth rate of MOFs is small and the nucleation rate is high, to produce a large-particle-size MOF, and thereby broaden the particle size distribution of the MOF and increasing the bulk density of the MOF. At the same time, since the present invention readily enables achieving a large supersaturation degree in the crystallization step, the production rate of the MOF per unit volume and per unit time can be made extremely high, enabling inexpensive production of MOFs.
The respective steps are described below.

[Reagent Introduction Step]

Since an MOF is a substance formed from a metal ion and an organic ligand, in this step, a metal ion and an organic ligand serving as raw materials for the MOF are first prepared. The metal ion is prepared in the form of an aqueous solution. The organic ligand is also prepared in the form of an aqueous solution.
The present production method is characterized in part by producing the MOF in water rather than in an organic solvent. Producing an MOF in water has advantages such as avoiding complexity of production equipment, providing high flexibility in production conditions, and reducing environmental impact.
As the metal ion, any suitable one may be used depending on the specific intended use of the MOF. When the MOF is used, for example, for gas storage or gas separation, the metal ion may be an aluminum ion, an iron ion, a copper ion, a zinc ion, a zirconium ion, a chromium ion, a cobalt ion, or the like.
Among the various metal ions listed above, an aluminum ion, an iron ion, a chromium ion, and a cobalt ion are generally hexacoordinated. A copper ion and a zinc ion are generally tetra-coordinated. A zinc ion may, depending on the type of solvent and the pH, form a cluster of four zinc nuclei and oxygen ions to form a hexavalent hexacoordinated ion. This four-nucleus zinc cluster ion is a type of zinc ion. A zirconium ion may be tetra-coordinated or octa-coordinated and, similar to a zinc ion, four tetracoordinate nuclei can form a cluster to become hexacoordinate.
Among the various metal ions listed above, it is preferable to use an aluminum ion, an iron ion, a four-nucleus zinc cluster, or a copper ion, since an MOF having excellent gas storage performance and gas separation performance can be easily obtained.
The aqueous solution of the metal ion can be obtained, for example, by dissolving a water-soluble metal compound in water. Examples of water-soluble metal compounds include water-soluble salts, halides, and hydroxides. Specifically, when the metal ion is, for example, an aluminum ion, aluminum sulfate or aluminum chloride can be used as the water-soluble metal compound. When the metal ion is, for example, an iron ion, iron(II) chloride, iron(III) chloride, or iron(II) sulfate can be used as the water-soluble metal compound. When the metal ion is, for example, a copper ion, copper(II) sulfate or copper(II) chloride can be used as the water-soluble metal compound. When the metal ion is, for example, a zinc ion, zinc sulfate can be used as the water-soluble metal compound. When the metal ion is, for example, a cobalt ion, cobalt(II) sulfate or cobalt(II) chloride can be used as the water-soluble metal compound.

The concentration of the metal ion in the aqueous solution of the metal ion is preferably 0.1 mol/L or more in terms of increasing the production rate per unit volume and per unit time. From the standpoint of further enhancing this advantage, the concentration of the metal ion in the aqueous solution of the metal ion is more preferably 0.15 mol/L or more, and even more preferably 0.3 mol/L or more.

Also, the concentration of the metal ion in the aqueous solution of the metal ion is preferably 1.8 mol/L or less since the concentration difference from the organic ligand does not become extreme. From the standpoint of further enhancing this advantage, the concentration of the metal ion in the aqueous solution of the metal ion is more preferably 1.0 mol/L or less, and even more preferably 0.9 mol/L or less.

The pH of the aqueous solution of the metal ion is not particularly limited as long as no precipitate containing the metal is formed in the liquid. When the pH of the aqueous solution of the organic ligand described later is basic, it is preferable to set the pH of the aqueous solution of the metal ion to 4 or less from the standpoint of neutralizing the pH of the liquid obtained by mixing those solutions.

The type of organic ligand used together with the metal ion to produce the MOF is not particularly limited as long as it is capable of coordinating to the metal ion. Examples of suitable organic ligands include nitrogen-donor ligands and oxygen-donor ligands.

Examples of nitrogen-donor ligands include imidazole, 2,2'-bipyridyl, 3,3'-bipyridyl, 4,4'-bipyridyl, 2,2'-bipyrazine, 2,2'-bipyrimidyl, and 1,4-di(4-pyridyl)benzene. These nitrogen-donor ligands may be used alone or in combination of two or more.

Meanwhile, examples of oxygen-donor ligands include anions of polybasic organic acids. The polybasic organic acid may be an aliphatic polybasic organic acid or an aromatic polybasic organic acid. Specific examples include dianions of dibasic organic acids such as oxalic acid, fumaric acid, terephthalic acid, phthalic acid, and isophthalic acid, as well as trianions of tribasic organic acids such as trimesic acid. A hydrogen atom bonded to an aromatic ring of the aromatic polybasic organic acid may be substituted by any of various functional groups such as a hydroxyl group, an amino group, or a nitro group. These oxygen-donor ligands may be used alone or in combination of two or more.

An organic ligand to be used may be selected appropriately in accordance with the specific intended use of the MOF. However, in terms of ease of obtaining an MOF having high bulk density, it is preferable to use an oxygen-donor ligand, and particularly preferable to use a dianion of a dibasic organic acid.

The concentration of the organic ligand in the aqueous solution is preferably 0.3 mol/L or more in terms of maximizing the production rate per unit volume and per unit time. To further enhance this advantage, the concentration of the organic ligand in the aqueous solution is more preferably 0.5 mol/L or more, and even more preferably 0.6 mol/L or more.

Also, the concentration of the organic ligand in the aqueous solution is preferably 0.9 mol/L or less since it is lower than or equal to the saturation solubility of the organic ligand. To further enhance this advantage, the concentration of the organic ligand in the aqueous solution is more preferably 0.85 mol/L or less, and even more preferably 0.8 mol/L or less.

The aqueous solution containing the organic ligand can be prepared, for example, by dissolving a polybasic organic acid in water or by dissolving a salt of the polybasic organic acid in water. When the solubility of the polybasic organic acid is low, for example, the polybasic organic acid can be dissolved more easily by adding the polybasic organic acid to water whose pH has been adjusted to 9 or higher using a basic substance such as sodium hydroxide (the aqueous solution in this case is an aqueous solution of a salt of the polybasic acid).

Depending on the type of polybasic acid or salt of the polybasic acid, the aqueous solution containing the organic ligand may contain a water-soluble organic solvent. The water-soluble organic solvent is mixed in order to improve the solubility of the polybasic acid or the salt of the polybasic acid. Examples of water-soluble organic solvents include: monohydric alcohols such as ethanol, methanol, and isopropyl alcohol; glycols such as ethylene glycol, diethylene glycol, polyethylene glycol, propylene glycol, dipropylene glycol, butylene glycol, and hexylene glycol; mono- or diethers of the foregoing glycols with lower alcohols such as methanol, ethanol, propanol, and butanol; esters of the foregoing glycols with lower fatty acids; and polyhydric alcohols such as glycerin and sorbitol. These water-soluble organic solvents may be used alone or in combination of two or more.

It is preferable that the aqueous solution containing the metal ion and the aqueous solution containing the organic ligand are added simultaneously into the reaction vessel, in terms of ease of obtaining an MOF having a high bulk density. Examples of modes of simultaneously adding the two aqueous solutions into the reaction vessel include: a mode in which the aqueous solution containing the metal ion and the aqueous solution containing the organic ligand are separately fed into a reaction vessel having a bottom portion and a wall portion continuous therewith; and a mode in which the aqueous solution containing the metal ion is fed from one end of a tubular reaction vessel and the aqueous solution containing the organic ligand is fed from the other end, causing the two aqueous solutions to meet in a counter-flow manner within the reaction vessel. From the standpoint of productivity of the MOF and ease of reaction control, it is preferable to adopt the mode in which the aqueous solution containing the metal ion and the aqueous solution containing the organic ligand are separately fed into a reaction vessel having a bottom portion and a wall portion continuous therewith.

It is preferable that the aqueous solution containing the metal ion and the aqueous solution containing the organic ligand are continuously added into the reaction vessel, in terms of ease of obtaining an MOF having a high bulk density. As used herein, the phrase "continuously added" includes not only adding the two aqueous solutions without interruption, but also adding them intermittently to an extent that can still be regarded as continuous. For example, when the two aqueous solutions are added intermittently with an interval of up to 600 seconds, such addition is included in "continuously added."

It is preferable that the aqueous solution containing the metal ion and the aqueous solution containing the organic ligand are added such that the number of moles of the metal ion in the aqueous solution containing the metal ion and the number of moles of the organic ligand in the aqueous solution containing the organic ligand are substantially stoichiometric for the intended MOF.

The combination of the metal ion and the organic ligand for producing the MOF can be selected appropriately depending on the specific intended use of the MOF.

Preferred combinations include combinations of hexacoordinated metal ions such as an aluminum ion, an iron ion, a chromium ion, a tetranuclear zinc cluster, and a cobalt ion with bidentate ligands such as a dibasic organic acid, and combinations of tetracoordinated metal ions such as a copper ion and a zinc ion with tridentate ligands such as a tribasic organic acid.

A specific example of the combination of a metal ion and an organic ligand is a combination of an aluminum ion as the metal ion and a fumaric acid dianion as the organic ligand. When adopting this combination, it is preferable to add 0.9 mol or more and 1.1 mol or less of the fumaric acid dianion per mol of the aluminum ion, more preferably 0.95 mol or more and 1.05 mol or less, and even more preferably 0.98 mol or more and 1.03 mol or less.

When the metal ion is an aluminum ion and the organic ligand is a terephthalic acid dianion, it is preferable to add 0.9 mol or more and 1.1 mol or less of the terephthalic acid dianion per mol of the aluminum ion, more preferably 0.95 mol or more and 1.05 mol or less, and even more preferably 0.98 mol or more and 1.03 mol or less.

When the metal ion is an iron(III) ion and the organic ligand is a terephthalic acid dianion, it is preferable to add 0.9 mol or more and 1.1 mol or less of the terephthalic acid dianion per mol of the iron(III) ion, more preferably 0.95 mol or more and 1.05 mol or less, and even more preferably 0.98 mol or more and 1.03 mol or less.

When the metal ion is a zinc(II) ion and the organic ligand is a fumaric acid dianion, it is preferable to add 0.9 mol or more and 1.1 mol or less of the fumaric acid dianion per 4 mol of the zinc(II) ion, more preferably 0.95 mol or more and 1.05 mol or less, and even more preferably 0.98 mol or more and 1.03 mol or less.

It is preferable that the aqueous solution containing the metal ion and the aqueous solution containing the organic ligand are introduced into the reaction vessel such that the pH of the reaction solution is maintained at a specific value.

For example, when producing an MOF formed from crystals of aluminum fumarate, it is preferable to maintain the pH of the reaction solution at 3 or more and 6 or less, more preferably at 3 or more and 5 or less, and even more preferably at 3 or more and 4.5 or less.

When producing an MOF formed from crystals of zinc fumarate, it is preferable to maintain the pH of the reaction solution at 5 or more and 9 or less, more preferably at 6 or more and 9 or less, and even more preferably at 6.5 or more and 8.5 or less.

When producing an MOF formed from crystals of aluminum terephthalate, it is preferable to maintain the pH of the reaction solution at 3 or more and 7 or less, more preferably at 3.5 or more and 7 or less, and even more preferably at 3.5 or more and 6 or less.

When producing an MOF formed from crystals of iron(III) terephthalate, it is preferable to maintain the pH of the reaction solution at 3 or more and 7 or less, more preferably at 4.5 or more and 6.5 or less, and even more preferably at 5 or more and 6 or less.

Since the present production method is a continuous method as mentioned above, it is advantageous in that the pH of the reaction solution is easily kept constant. In contrast, in a batch-type production method, the pH of the reaction solution is more likely to change as the reaction proceeds, making it difficult to maintain an optimal pH value from the start of the reaction to the end of the reaction.

To keep the pH of the reaction solution within the above preferred numerical range, it is preferable to feed a basic substance such as sodium hydroxide into the reaction vessel simultaneously with feeding the metal ion and the organic ligand into the reaction vessel. To achieve this, for example, the aqueous solution containing the organic ligand may contain the basic substance.

From the standpoint of appropriately controlling the pH of the reaction solution and successfully obtaining a desired MOF, it is preferable that the molar ratio of the basic substance to the organic ligand fed into the reaction vessel takes a specific value.

For example, when the organic ligand is fumaric acid, the metal ion is an aluminum ion, and the basic substance is sodium hydroxide, the molar ratio (basic substance / organic ligand) is preferably 2.5 or more and 4.5 or less, more preferably 2.6 or more and 4.4 or less, and even more preferably 2.7 or more and 3.8 or less.

When the organic ligand is fumaric acid, the metal ion is a zinc ion, and the basic substance is sodium hydroxide, the molar ratio (basic substance / organic ligand) is preferably 7 or more and 10 or less, more preferably 7.5 or more and 9.5 or less, and even more preferably 7.5 or more and 8.5 or less.

When the organic ligand is terephthalic acid, the metal ion is an aluminum ion, and the basic substance is sodium hydroxide, the molar ratio (basic substance / organic ligand) is preferably 2.5 or more and 4 or less, more preferably 2.6 or more and 4 or less, and even more preferably 2.8 or more and 3.5 or less.

When the organic ligand is terephthalic acid, the metal ion is an iron(III) ion, and the basic substance is sodium hydroxide, the molar ratio (basic substance / organic ligand) is preferably 2 or more and 4 or less, more preferably 2.4 or more and 3.8 or less, and even more preferably 2.6 or more and 3.3 or less.

Note that the number of moles of the basic substance used for calculating the above molar ratio (basic substance / organic ligand) also includes the number of moles of the basic substance required to neutralize the organic ligand.

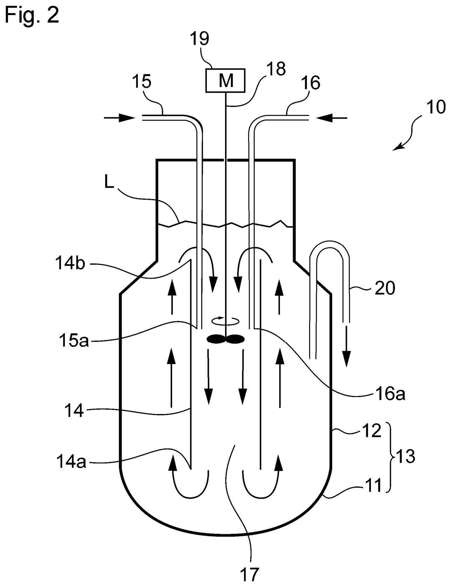

Fig. 2 schematically shows an example of a reaction apparatus suitably used in the present production method. However, this example is not limiting if an apparatus includes four steps equivalent to those of the present invention. A reaction apparatus 10 shown in the figure is a crystallizer called a draft tube baffle (hereinafter also referred to as "DTB") type. The reaction apparatus 10 includes a reaction vessel 13 having a bottom portion 11 and a wall portion 12 continuous with the bottom portion 11. A draft tube 14 having a tubular shape is disposed in the reaction vessel 13. The draft tube 14 is disposed in the reaction vessel 13 such that its axial direction is parallel to the depth direction of the reaction vessel 13. The lateral cross-sectional shape of the draft tube 14 may be, for example, circular, elliptical, or polygonal. Upper and lower ends of the draft tube 14 are open. A lower end 14a of the draft tube 14 is spaced apart at a predetermined distance from the bottom portion of the reaction vessel 13.

The reaction apparatus 10 includes a first feed pipe 15 for feeding the aqueous solution containing the metal ion into the reaction vessel 13. The reaction apparatus 10 also includes a second feed pipe 16 for feeding the aqueous solution containing the organic ligand into the reaction vessel 13. Both the first feed pipe 15 and the second feed pipe 16 are disposed inside the draft tube 14. Lower ends 15a and 16a of the first feed pipe 15 and the second feed pipe 16 are disposed so as to be located above the lower end 14a of the draft tube 14. A region around the lower ends 15a and 16a of the first feed pipe 15 and the second feed pipe 16 corresponds to the aforementioned reagent introduction zone.

The reaction apparatus 10 includes a liquid stirring means. Examples of the stirring means include stirring by rotation of an impeller, stirring by circulating the liquid using a pump, and stirring by introducing bubbles. Fig. 2 shows a stirring means using the rotation of an impeller 17. The impeller 17 is connected to a lower end of a drive shaft 18, and an upper end of the drive shaft 18 is connected to a drive source 19 such as a motor installed outside the reaction vessel 13. When the drive shaft 18 is driven by the drive source 19, the drive shaft 18 and the impeller 17 connected thereto rotate about the drive shaft, thereby creating a downward or upward flow. A region around the impeller 17 corresponds to the aforementioned crystallization zone. In this case, it is preferable that the sum of the volumes of the reagent introduction zone and the crystallization zone of the reaction apparatus 10 is 30% or less of the volume of the crystallizer, in terms of ease of obtaining coarse crystals by increasing the number of times of circulation. From the standpoint of further enhancing this advantage, the sum of the volumes of the reagent introduction zone and the crystallization zone is preferably 10% or more and 30% or less of the volume of the crystallizer, more preferably 15% or more and 30% or less, and even more preferably 20% or more and 30% or less.

[Crystallization Step]

In this step, the aqueous solution containing the metal ion and the aqueous solution containing the organic ligand that have been fed into the reaction vessel are mixed to react the metal ion and the organic ligand to form crystals of an intended MOF. This reaction will be described with reference to Fig. 2.

It is preferable that the reaction between the metal ion and the organic ligand in this step is carried out such that the reaction product formed in the mixed solution of the two aqueous solutions is maintained in a supersaturated state. Thus, the MOF as the reaction product achieves a high bulk density.

As used herein, the term "supersaturated state" refers to a state in which the concentration of the MOF as the reaction product in the reaction solution is higher than its saturation solubility.

In order to make the MOF in a supersaturated state, the present production method uses the reaction apparatus 10 constituted by the above-described DTB-type crystallizer shown in Fig. 2. In the reaction apparatus 10 in Fig. 2, the aqueous solution containing the metal ion and the aqueous solution containing the organic ligand are fed into the draft tube 14 through the first feed pipe 15 and the second feed pipe 16 that are disposed within the draft tube 14, and the two aqueous solutions are mixed to form a reaction solution. At this time, a downward flow is caused in the reaction solution in the draft tube 14 by controlling the rotation direction of the impeller 17. The aforementioned supersaturated state tends to occur within the draft tube 14. Particularly, it tends to occur between the impeller 17 and the lower ends 15a and 16a of the first feed pipe 15 and the second feed pipe 16. The supersaturated state may occur locally or throughout the entire reaction solution.

When feeding the aqueous solution containing the metal ion and the aqueous solution containing the organic ligand into the reaction vessel 13, one or both of the two aqueous solutions may be fed in a heated state, from the standpoint of promoting the reaction. The heating temperature may be, for example, 40°C or higher, preferably 60°C or higher, and more preferably 70°C or higher. Also, the heating temperature may be 95°C or lower, preferably 90°C or lower, and more preferably 85°C or lower.

Regardless of whether the aqueous solution containing the metal ion and/or the aqueous solution containing the organic ligand is heated, the reaction can be carried out in an open system, i.e., under atmospheric pressure. This is advantageous in simplifying the reaction apparatus 10 and facilitating control of the reaction. However, the present production method may also be carried out in a closed system, e.g., under an autogenous pressure exceeding 1 atmospheric pressure.

While the reaction solution flows downward in the draft tube 14, the metal ion and the organic ligand react to form crystal nuclei of the MOF, and crystals grow around the crystal nuclei. The formed MOF crystals, together with the reaction solution, move from the lower end 14a of the draft tube 14 toward the bottom portion 11 of the reaction vessel 13, and upon the MOF crystals colliding with the bottom portion 11, the flow direction turns upward.

The reaction solution containing the MOF crystals then flows upward along the outer surface of the draft tube 14, and reaches the upper end 14b of the draft tube 14. A region in which this flow occurs corresponds to the aforementioned circulation zone. The liquid circulating in the crystallizer is a slurry that is formed from the aqueous solution containing the metal ion and the aqueous solution containing the organic ligand ion and in which crystals of the formed metal-organic framework are suspended. These liquids are completely mixed together and circulate in the crystallizer.

As mentioned above, a downward flow is caused in the draft tube 14. Thus, the reaction solution that has reached the upper end 14b of the draft tube 14 (this reaction solution contains the MOF) is drawn into the draft tube 14 and flows downward in the draft tube 14. Since the aqueous solution containing the metal ion and the aqueous solution containing the organic ligand are fed into the draft tube 14 through the first feed pipe 15 and the second feed pipe 16 as mentioned above, the MOF contained in the reaction solution reacts with the newly fed metal ion and organic ligand, thereby promoting the growth of the MOF crystals and aggregation of the crystals, and increasing the particle size of the MOF.

Thus, according to the present production method, the reaction solution circulates inside and outside the draft tube 14 serving as a boundary, resulting in simultaneous and continuous formation of MOF crystal nuclei and crystal growth. As a result, an MOF having a broad particle size distribution, ranging from particles with large particle sizes to those with small particle sizes, is formed. In an MOF having a broad particle size distribution, crystals with small particle sizes readily fill spaces between crystals with large particle sizes. Thus, the MOF achieves a high bulk density.

The degree of circulation of the reaction solution can be controlled by the rotational speed of the impeller 17. Generally, the higher the rotational speed of the impeller 17 is, the higher the circulation speed of the reaction solution is.

Note that, in order to circulate the reaction solution along the up-down direction of the reaction vessel 13, it is preferable to adjust the amount of reaction solution such that a liquid surface L is positioned above the upper end 14b of the draft tube 14.

From the standpoint of successfully obtaining an MOF having a broad particle size distribution, it is preferable in this step to adjust the nucleation rate at which crystal nuclei of the MOF are formed by the reaction between the metal ion and the organic ligand. From this standpoint, the nucleation rate of the MOF crystals is preferably $1 \times 10^5$ nuclei/(h·m³) or more, more preferably $5 \times 10^5$ nuclei/(h·m³) or more, and even more preferably $1 \times 10^6$ nuclei/(h·m³) or more. Also, the nucleation rate of the MOF crystals is preferably $1 \times 10^{16}$ nuclei/(h·m³) or less, more preferably $5 \times 10^{15}$ nuclei/(h·m³) or less, and even more preferably $1 \times 10^{14}$ nuclei/(h·m³) or less.

The nucleation rate of the MOF crystals can be controlled by adjusting, for example, the rotational speed of the impeller 17, the diameter of the draft tube 14, and the concentrations and feed rates of the aqueous solution containing the metal ion and the aqueous solution containing the organic ligand.

The nucleation rate of the MOF crystals is measured by the following method.

It is known that, once a steady state is established in a continuous crystallizer, the production rate P/ρcV', the nucleation rate Fv', the linear growth rate $(dl/d\theta)_{av}$, the particle size $l_p^*$, the particle size distribution m, and the crystal suspension density $(1-\varepsilon_i)$ satisfy the relationships shown in the following equations (Nippon Chemical Industrial Co., Ltd., Creative, 1, No. 5 (2004); Internet <URL:https://www.nippon-chem.co.jp/dcms_media/other/cre2004-2.pdf>).

In the equations below, V' represents the apparatus volume, $\rho_c$ represents the density of the crystals, $k_a$ represents the surface area-shape factor of the crystals, and $k_v$ represents the volume-shape factor of the crystals. m represents the slope of a particle size distribution line plotted on a Rosin-Rammler diagram, and $l_p^*$ represents the particle size characteristic value obtained by plotting product crystals on a Rosin-Rammler diagram.

[Formula 1]

$$P/\rho_c V' = l_3^{-1}(1-\varepsilon_i)\left(\frac{dl}{d\theta}\right)_{av}\frac{k_a\int_0^{x_{max}}(x^2\exp{-x^m})dx}{k_v\int_0^{x_{max}}(x^3\exp{-x^m})dx}$$

(Equation 1)

$$= F_v'\, k_v\, l_3^3 \int_0^{x_{max}}(mx^{m+2}\exp{-x^m})dx$$

(Equation 2)

Here, since there are six variables and two equations, the degrees of freedom are four. Therefore, if four of these variables can be measured, the remaining two variables can be calculated. The production rate $P/\rho cV'$ can be measured from the mass of the crystals obtained from the crystallizer. The particle size l and the particle size distribution m can be measured by microscopic observation of the crystals or by a particle size distribution measuring device. The suspension density $(1-\varepsilon_i)$ can be measured by sampling the slurry in the crystallizer and measuring the volume ratio between the slurry and the crystals. As a result, the nucleation rate and the linear growth rate can be measured using the two equations, equivalently to directly measured values.

In this step, in addition to or instead of controlling the nucleation rate of the MOF crystals as described above, controlling the linear growth rate of the MOF crystals is also advantageous from the standpoint of successfully obtaining an MOF having a broad particle size distribution. From this standpoint, the linear growth rate of the MOF crystals is preferably 1 $\mu$m/h or more, more preferably 10 $\mu$m/h or more, and even more preferably 80 $\mu$m/h or more. Also, the linear growth rate of the MOF crystals is preferably 200 $\mu$m/h or less, more preferably 150 $\mu$m/h or less, and even more preferably 100 $\mu$m/h or less.

The linear growth rate of the MOF crystals can be controlled by adjusting, for example, the rotational speed of the impeller 17, the diameter of the draft tube 14, and the concentrations and feed rates of the aqueous solution containing the metal ion and the aqueous solution containing the organic ligand.

The linear growth rate of the MOF crystals is measured by the same method as the above-described measurement of the nucleation rate.

[Crystal Recovery Step]

In this step, the MOF crystals precipitated in the reaction solution are removed from the reaction vessel 13. The crystals are removed using an outlet pipe 20 provided in the reaction apparatus 10. The outlet pipe 20 has one end located inside the reaction vessel 13, and the other end located outside the reaction vessel 13. Although not shown in the figure, the outlet pipe 20 may be equipped with a pump and/or an on-off valve for removing the reaction solution. A region in which the crystals are removed by the outlet pipe 20 corresponds to the aforementioned recovery zone.

The MOF crystals contained in the reaction solution are withdrawn to the outside of the reaction vessel 13 through the outlet pipe 20 together with the reaction solution. It is preferable that the reaction solution containing the MOF crystals is withdrawn continuously or at predetermined time intervals. In the case where the reaction solution is withdrawn at predetermined time intervals, the interval may be constant or non-constant. In either case, the withdrawal interval is preferably 0.5 minutes or more and 10 minutes or less, depending on the capacity of the reaction vessel 13 and the MOF production volume, from the standpoint of maintaining a constant volume of the crystallizer. From the standpoint of further enhancing this advantage, the withdrawal interval is more preferably 1 minute or more and 5 minutes or less, and even more preferably 1 minute or more and 2 minutes or less.

It is desirable that the withdrawal amount of reaction solution is controlled such that the MOF formation reaction in the reaction vessel 13 is in a steady state. Specifically, it is desirable to withdraw the reaction solution such that the amount of MOF formed in the reaction vessel 13 and the amount of MOF withdrawn through the outlet pipe 20 are balanced.

To produce an MOF having a large crystal particle size, it is advantageous to increase the number of times that the crystals pass through the reagent introduction zone and the crystallization zone. The number of times X [number of times/min] that a crystal passes through the zones is a value obtained by dividing a discharge flow rate Q [$m^3$/min] of the impeller 17 by the total volume V [$m^3$] of the reagent introduction zone A and the crystallization zone B.

$$X = Q / V \qquad \text{(Equation 3)}$$

Each time a crystal passes through the zones, it grows by the linear growth amount G [$\mu$m] corresponding to the supersaturation degree and the residence time in the zones, and the particle diameter $L_{total}$ [$\mu$m/min] is a value obtained by multiplying this linear growth amount by the number of times X that the crystal passes.

$$L_{total} = G \times X \qquad \text{(Equation 4)}$$

The particle diameter $L_{total}$ is proportional to the number of times that the crystal passes. Thus, the longer the overall residence time in the crystallizer is, the larger the particle size becomes. However, since nucleation occurs simultaneously with crystal growth, the crystal particle size distribution becomes broader, and the average crystal particle size over the entire apparatus does not necessarily correlate with the overall residence time. In any case, the longer the residence time over the entire apparatus is, the more easily coarse crystals are obtained. Thus, the longer the residence time over the entire apparatus is, the broader the particle size distribution becomes.

As described above, when withdrawing the reaction solution, it is also advantageous to control the residence time of the reaction solution from the standpoint of successfully obtaining an MOF having a broad particle size distribution. From this standpoint, it is preferable to withdraw MOF crystals from the reaction vessel 13 such that the residence time in the crystallizer, including all of the reagent introduction zone, the crystallization zone, the circulation zone, and the recovery zone, is 3 minutes or more. Particularly, it is preferable to withdraw the MOF crystals from the reaction vessel 13 such that the residence time is 5 minutes or more, and even more preferably 10 minutes or more.

From the same standpoint, it is preferable to withdraw the MOF crystals from the reaction vessel 13 such that the residence time is 30 minutes or less. Particularly, it is preferable to withdraw the MOF crystals from the reaction vessel 13 such that the residence time is 25 minutes or less, and even more preferably 20 minutes or less.

In the MOF reaction, the residence time over the entire apparatus is determined by dividing the volume of the entire apparatus by the sum of the flow rates of the metal component and the organic ligand component in the double decomposition reaction.

[Washing Step]

The MOF crystals withdrawn from the reaction vessel 13 are subjected to a washing step as necessary. In the washing step, water is mainly used for washing. For example, the reaction solution containing the MOF crystals is withdrawn from the reaction vessel 13, and the reaction solution is placed in a nutsche filter (Buchner funnel) or the like to separate liquid from the MOF crystals by filtration. Thus, cake-like MOF crystals are obtained, and impurities can be removed from these crystals by subsequently washing them with water. Alternatively, the MOF crystals can be washed by a hydraulic elutriation method. As another method, a washing method using a liquid cyclone can also be adopted.

Particularly, when the metal-organic framework produced by the above-described method is subjected to the following continuous washing method, the amount of washing liquid can be reduced, which is advantageous. This continuous washing method includes a washing step of washing the metal-organic framework with a washing liquid to remove a counteranion for dissolving the metal ion constituting the metal-organic framework and a countercation for dissolving the organic ligand. Examples of the washing liquid include water and mixed solvents of water and a water-soluble organic solvent. Examples of the water-soluble organic solvent include water-soluble lower aliphatic alcohols. Preferred examples of the water-soluble lower aliphatic alcohol include aliphatic monohydric alcohols having 1 to 4 carbon atoms, specifically methanol, ethanol, and isopropyl alcohol.

The washing step preferably includes a first washing step, a solid-liquid separation step, and a second washing step. In these steps, it is preferable that the liquid subjected to washing in the first washing step is separated into a solid component and a liquid component in the solid-liquid separation step, that the solid component is further washed in the second washing step, and that the liquid component is used as part of the washing liquid in the first washing step. This makes it possible to reduce the amount of washing liquid, as mentioned above. Particularly, by repeating the washing step and the solid-liquid separation step multiple times and using the liquid component obtained in the solid-liquid separation step as part of the washing liquid in the preceding washing step, the amount of washing liquid can be further reduced.

The means for separating the solid component and liquid component in the solid-liquid separation step is not particularly limited. For example, the solid component and the liquid component can be efficiently separated by using centrifugal force.

[MOF Obtained by the Present Production Method]

The MOF obtained by the present production method typically has a composition formula represented by a general formula $ML_xA_y$.

In the general formula, M represents a metal ion, L represents an organic ligand, and A represents an anion other than the organic ligand represented by L. Details of M and L are as described above. The combination of M and L may be appropriately selected in accordance with the specific intended use of the MOF. For example, a fumaric acid dianion or a terephthalic acid dianion can be selected as M, and an aluminum ion, a zinc ion, or an iron(III) ion may be selected as L.

In the above general formula, the anion represented by A is of one or more types selected from a hydroxide ion, a sulfate ion, a chloride ion, and an oxide ion ($O^{2-}$).

In the above general formula, x represents the number of moles of the organic ligand L per mole of the metal ion M. For example, when M is an aluminum ion or an iron(III) ion and L is a fumaric acid dianion or a terephthalic acid dianion, x is preferably 0.85 or more and 1.15 or less, more preferably 0.88 or more and 1.12 or less, and even more preferably 0.9 or more and 1.1 or less.

When M is a zinc ion and L is a terephthalic acid dianion, x is preferably 0.6 to 0.9, more preferably 0.65 to 0.85, and even more preferably 0.7 to 0.8.

When M is a zinc ion and L is a fumaric acid dianion, x is preferably 0.20 or more and 0.30 or less, more preferably 0.22 or more and 0.28 or less, even more preferably 0.23 or more and 0.27 or less, and particularly preferably 0.24 or more and 0.26 or less.

In the above general formula, y represents the number of moles of the anion A per mole of the metal ion M. For example, when M is an aluminum ion, L is a fumaric acid dianion or a terephthalic acid dianion, and A is a hydroxide ion, y is preferably 0.7 or more and 1.2 or less, more preferably 0.8 or more and 1.2 or less, and even more preferably 0.9 or more and 1.1 or less.

When M is an iron(III) ion, L is a fumaric acid dianion or a terephthalic acid dianion, and A is a hydroxide ion, y is preferably 0.8 or more and 1.2 or less, more preferably 0.88 or more and 1.12 or less, and even more preferably 0.9 or more and 1.1 or less.

When M is a zinc ion, L is a terephthalic acid dianion, and A is an oxide ion, assuming x = 3/4, y is preferably 0.20 or more and 0.30 or less, more preferably 0.22 or more and 0.28 or less, even more preferably 0.23 or more and 0.27 or less, and particularly preferably 0.24 or more and 0.26 or less.

When M is a zinc ion, L is a fumaric acid dianion, and A is a hydroxide ion and an oxide ion, the MOF is represented by a composition formula $ZnL_x(OH)_{y1}O_{y2}$ (where L represents the fumaric acid dianion). In this case, assuming x = 1/4, y1 is preferably 0.8 or more and 1.2 or less, more preferably 0.88 or more and 1.15 or less, and even more preferably 0.9 or more and 1.1 or less. y2 is preferably 0.20 or more and 0.30 or less, more preferably 0.22 or more and 0.28 or less, even more preferably 0.23 or more and 0.27 or less, and particularly preferably 0.24 or more and 0.26 or less.

According to the present production method, it is possible to easily obtain an MOF having a broad particle size distribution ranging from a large particle size to a small particle size and having high crystallinity. It is possible to confirm that the MOF obtained by this method has a broad particle size distribution through measurement using a particle size distribution measuring device, or it can be evaluated by tap density. When evaluating the particle size distribution of the MOF based on the tap density, the larger the tap density is, it can be evaluated that the particle size distribution of the MOF is broader. The tap density is a mass per unit volume when powder that has naturally fallen is filled into a fixed container, thereafter the container is impacted with a tap, and the sample volume stops changing. The tap density can be measured in accordance with JIS K 5101-12-2:2004. Specifically, the tap density may be measured, for example, by using DUAL AUTOTAP (manufactured by Yuasa Ionics Co., Ltd.). The tap density of the MOF measured by this method is preferably 0.3 g/cm³ or more and 0.9 g/cm³ or less, more preferably 0.4 g/cm³ or more and 0.9 g/cm³ or less, even more preferably 0.5 g/cm³ or more and 0.8 g/cm³ or less, and further preferably 0.6 g/cm³ or more and 0.7 g/cm³ or less. Thus, since the MOF obtained by this production method has a high tap density, when it is used as an adsorbent for carbon dioxide, for example, the adsorption amount of carbon dioxide per unit volume becomes high.

In addition to having a broad particle size distribution, the MOF obtained by the present production method is also characterized by having a large average particle size. Specifically, the average particle size of the MOF obtained by the present production method is preferably 10 μm or more and 150 μm or less, more preferably 20 μm or more and 100 μm or less, and even more preferably 30 μm or more and 100 μm or less.

The average particle size of the MOF is measured by the following method.

When, as in the present invention, the number of MOF particles having particle sizes of 1 μm or more is predominant, the particle size distribution can be measured by performing image analysis using an optical microscope VHX-7000 manufactured by Keyence Corporation. In addition, measurement can also be performed using a laser scattering particle size distribution analyzer LA-960 manufactured by HORIBA, Ltd. Although the absolute values of particle size cannot be compared due to the different evaluation criteria of these instruments, the particle size can be evaluated as to which particles are larger or smaller without problem, based on the average particle size measured by the same method.

In any case, to calculate the nucleation rate and linear growth rate, it is useful to obtain not only the so-called average particle size (domain size), but also the particle size characteristic number L* and the slope m of the Risin-Rammler distribution.

Further, the MOF obtained by the present production method is also characterized in that, despite having a large average particle size, it has a large BET specific surface area. Specifically, the BET specific surface area of the MOF obtained by the present production method is preferably 740 m²/g or more and 1200 m²/g or less, more preferably 800 m²/g or more and 1150 m$^2$/g or less, and even more preferably 900 m$^2$/g or more and 1150 m$^2$/g or less.

The BET specific surface area of the MOF is measured by the following method.

A sample of 10 mg to 20 mg was introduced into a measurement container, and a pretreatment step was performed in which the sample was held at 573 K for one hour under vacuum evacuation to remove adhered substances such as water. After evacuating the inside of the measurement container at 77 K, nitrogen was gradually introduced into the container to adsorb the nitrogen onto the MOF. The amount of nitrogen adsorbed onto the MOF was measured, and subsequently the amount of nitrogen desorbed was measured by reducing the pressure inside the container. The relationship between the adsorption/desorption amounts of nitrogen and the change in pressure was thereby examined, and the BET specific surface area was obtained.

Furthermore, the MOF obtained by the present production method is also characterized by having a high crystallinity degree. Specifically, the crystallinity degree of the MOF obtained by the present production method takes a high value of 100% or more, particularly 150% or more, and especially 200% or more.

The crystallinity degree of the MOF is measured by the following method.

The MOF is analyzed by powder X-ray analysis, and the height of the highest peak, i.e., a first peak, appearing near approximately $2\theta=10°$ is obtained. The ratio of the height of the corresponding peak is obtained with respect to a commercially available MOF manufactured by Sigma-Aldrich (CAS No.: 1370461-06-5), and the crystallinity degree can be obtained by setting a reference height as 100%.

Also, due to the high crystallinity degree, the MOF obtained by the present production method exhibits small changes in the diffraction peak angle and the diffraction peak height observed by XRD, even after repeated adsorption and desorption of water.

[Gas Adsorption Material]

The MOF obtained by the present production method is suitably used as a gas adsorption material, for example. Accordingly, the present invention provides the use of the MOF obtained by the present production method as a gas adsorption material.

In particular, the present invention provides the use, as a gas adsorption material, of a metal-organic framework formed from aluminum fumarate crystals and having a tap density of 0.3 g/mL or more and 0.9 g/mL or less.

The gas adsorption material is formed, for example, from a granulated body of a mixture of an MOF serving as a gas adsorption component and a phenolic resin serving as a shape-retaining component.

Preferably, the gas adsorption material includes:

a step A of mixing an MOF and a thermosetting phenolic resin to obtain a mixture;
a step B of adding water to the mixture and performing tumbling granulation to obtain granulated materials;
a step C of separating the granulated materials that have grown to a predetermined particle size, in accordance with their particle sizes; and
a step D of again subjecting the granulated materials that do not reach the predetermined particle size to the granulation step, heating the granulated materials that have reached the predetermined particle size at a first temperature to remove the water, and further heating them to a second temperature to cure the thermosetting phenolic resin.

The type of thermosetting phenolic resin used in the step A is not particularly limited, and any thermosetting phenolic resin known to date may be used.

When water is added in the step B, the water acts as a coagulant, and the granulated materials grow in a snowball-like manner during tumbling granulation, enabling efficient production of the granulated materials. From this standpoint, it is preferable to add water in atomized form. For atomizing the water, a mist-generating device such as a sprayer may be used.

In the step C, for example, a sieving operation may be performed in order to separate the granulated materials in accordance with the particle size.

The first temperature adopted in the step D may be any temperature that enables the removal of water contained in the granulated materials, and may be set to, for example, 80°C or higher and 130°C or lower. The second temperature for curing the thermosetting phenolic resin is higher than the first temperature and depends on the type of thermosetting phenolic resin, but may generally be set to higher than 130°C and 200°C or less.

In order to achieve a strength for withstanding practical use, the gas adsorption material obtained in this manner preferably has a crushing strength of 20 N or more, more preferably 30 N or more, and even more preferably 50 N or more.

From the same standpoint, the gas adsorption material preferably has a rotational strength of 3% or less when evaluated using a rolling device, more preferably 2% or less, and even more preferably 1% or less.

The crushing strength of the gas adsorption material is measured in accordance with the Association of Powder Process Industry and Engineering, Japan Standard G001: Method for measuring crushing strength of granulated materials (= JIS Z 8841:1993).

The rotational strength of the gas adsorption material is measured by the Association of Powder Process Industry and Engineering, Japan Standard G002: Method for measuring rotational strength of granulated materials (= JIS Z 8841:1993)."

Although the present invention has been described above based on the preferred embodiment, the present invention is not limited to the above embodiment. For example, although in the above embodiment a downward flow is imparted to the reaction solution in the draft tube 14 by rotating the impeller 17, the reaction may instead be carried out by imparting an upward flow to the reaction solution in the draft tube 14.

Examples

Hereinafter, the present invention will be described in further detail based on examples. However, the scope of the present invention is not limited to the examples given below.

[Example 1]

In this example, an MOF formed from aluminum fumarate was produced continuously using the reaction apparatus 10 shown in Fig. 2.

An aluminum sulfate aqueous solution having a pH of 3.1 and a concentration of 0.8 mol/L was obtained by dissolving aluminum sulfate in water. Also, a fumaric acid aqueous solution having a pH of 14.1 and a concentration of 0.75 mol/L was obtained by dissolving fumaric acid and 3 molar equivalents of sodium hydroxide relative to the fumaric acid in water.

The aluminum sulfate aqueous solution and the fumaric acid aqueous solution were simultaneously and continuously fed into the reaction vessel 13 having a volume of 3.3 L. The feed rate of the aluminum sulfate aqueous solution was 0.07 L/min. The feed rate of the fumaric acid aqueous solution was 0.15 L/min. The liquid temperatures of both the aluminum sulfate aqueous solution and the fumaric acid aqueous solution were set to 80°C. The pH of the reaction solution remained substantially constant at 3.6.

The reaction between the aluminum ion and the fumarate ion was controlled by the rotational speed of the impeller 17. The nucleation rate and the linear growth rate measured by the above method were as shown in Table 1 below. The MOF formed in the reaction vessel 13 by the reaction was continuously withdrawn to the outside of the reaction vessel 13 through the outlet pipe 20 along with the reaction solution. At this time, the withdrawal amount of MOF was adjusted to balance the amount of MOF formed in the reaction vessel 13. The residence time of the reaction solution measured by the above method was as shown in Table 2 below. Other conditions are also as shown in Table 2.

Further, the tap density, average particle size, BET specific surface area, and crystallinity degree of the obtained MOF were measured by the above-described method. The results are shown in Table 2 below.

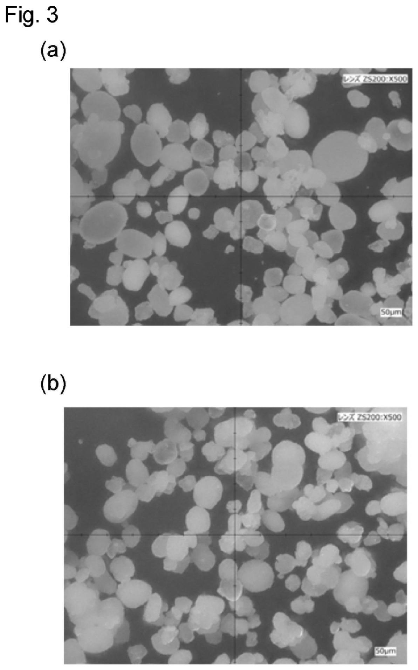

Figs. 3(a) and (b) show optical microscope photographs of the obtained MOF crystals. The crystals were translucent. The surfaces of the crystals with small particle sizes were somewhat uneven, while the overall shape of each crystal was a rounded ellipsoid. To determine a volume-shape factor and a surface area-shape factor, the crystals were approximately regarded as spherical, and those factors were calculated using the above-described method.

The production rate $P/\rho cV'$ was calculated by measuring the mass of the MOF crystals obtained from the crystallizer, based on the density of the crystals and the volume of the crystallizer. The particle size l and the particle size distribution m were measured by microscopic observation of the crystals, or by a particle size distribution measuring device. The suspension density $(1-\varepsilon_i)$ was measured by sampling the slurry in the crystallizer and measuring the volume ratio between the slurry and the crystals. The nucleation rate and the linear growth rate were calculated using Equations 1 and 2, and are shown in Table 1. Table 1 contains the results of experiments conducted under various conditions. Runs 1 to 3 show the results obtained with a reaction vessel volume of 3.3 L and various residence times. Runs 4 to 9 show the results obtained with a reaction vessel volume of 10 L and various residence times. In Runs 8 and 9, the concentrations of fumaric acid and aluminum sulfate were changed.

The sum of the volumes of the reagent introduction zone and the crystallization zone in Runs 1 to 3 was 18% of the volume of the crystallizer.

The sum of the volumes of the reagent introduction zone and the crystallization zone in Runs 4 to 9 was 25% of the volume of the crystallizer.

Table 2 shows a summary of the results in Table 1. Values in Table 2 are average values of Runs 1 to 9.

[Example 2]

In this example, an MOF formed from aluminum terephthalate was produced continuously using the reaction apparatus 10 shown in Fig. 2.

An aluminum sulfate aqueous solution having a pH of 3.1 and a concentration of 0.8 mol/L was obtained by dissolving aluminum sulfate in water. Also, a terephthalic acid aqueous solution having a pH of 14.2 and a concentration of 0.75 mol/L was obtained by dissolving terephthalic acid and sodium hydroxide in water.

The aluminum sulfate aqueous solution and the terephthalic acid aqueous solution were simultaneously and continuously fed into the reaction vessel 13 having a volume of 10 L. The feed rate of the aluminum sulfate aqueous solution was 0.21 L/min. The feed rate of the fumaric acid aqueous solution was 0.45 L/min. The liquid temperature of both the aluminum sulfate aqueous solution and the terephthalic acid aqueous solution was set to 80°C. The pH of the reaction solution remained substantially constant at 5.8.

The reaction between the aluminum ion and the terephthalate ion was controlled by the rotational speed of the impeller 17. The nucleation rate and the linear growth rate measured by the above method were as shown in Table 1 below. The MOF formed in the reaction vessel 13 by the reaction was continuously withdrawn to the outside of the reaction vessel 13 through the outlet pipe 20 along with the reaction solution. At this time, the withdrawal amount of MOF was finely adjusted to balance the amount of MOF formed in the reaction vessel 13 and maintain the liquid level in the crystallizer constant. The residence time of the reaction solution measured by the above method was as shown in Table 2 below.

The tap density, average particle size, BET specific surface area, and crystallinity degree of the obtained MOF were measured by the above method. The results are shown in Table 2 below.

In this example, the sum of the volumes of the reagent introduction zone and the crystallization zone was 25% of the volume of the crystallizer.

[Example 3]

In this example, an MOF formed from zinc fumarate was produced continuously using the reaction apparatus 10 shown in Fig. 2.

A zinc chloride aqueous solution having a pH of 4.1 and a concentration of 0.4 mol/L was obtained by dissolving zinc sulfate in water. Also, a fumaric acid aqueous solution having a pH of 12.6 and a concentration of 0.1 mol/L was obtained by dissolving fumaric acid and 8 molar equivalents of sodium hydroxide relative to the fumaric acid in water.

The zinc chloride aqueous solution and the fumaric acid aqueous solution were simultaneously and continuously fed into the reaction vessel 13 having a volume of 10 L. The feed rate of the zinc chloride aqueous solution was 0.3 L/min. The feed rate of the fumaric acid aqueous solution was 0.3 L/min. The liquid temperature of both the zinc chloride aqueous solution and the fumaric acid aqueous solution was set to 80°C. The pH of the reaction solution remained substantially constant at 7.5.

The reaction between the zinc ion and the fumarate ion was controlled by the rotational speed of the impeller 17. The nucleation rate and the linear growth rate measured by the above method were as shown in Table 2 below. The MOF formed in the reaction vessel 13 by the reaction was continuously withdrawn to the outside of the reaction vessel 13 through the outlet pipe 20 along with the reaction solution. At this time, the withdrawal amount of MOF was adjusted to balance the amount of MOF formed in the reaction vessel 13. The residence time of the reaction solution measured by the above method was as shown in Table 2 below.

The tap density, average particle size, BET specific surface area, and crystallinity degree of the obtained MOF were measured by the above method. The results are shown in Table 2 below.

In this example, the sum of the volumes of the reagent introduction zone and the crystallization zone was 25% of the volume of the crystallizer.

[Reference Example 1]

In this reference example, an MOF formed from aluminum fumarate was produced by a batch method.

An aluminum sulfate aqueous solution having a pH of 3 and a concentration of 0.32 mol/L was obtained by dissolving aluminum sulfate in water. A fumaric acid aqueous solution having a pH of 14 and a concentration of 0.63 mol/L was obtained by dissolving fumaric acid and sodium hydroxide in water.

1 L of the aluminum sulfate aqueous solution was placed in a reaction vessel having a volume of 3.3 L and stirred with stirring blades. 1.1 L of the fumaric acid aqueous solution was added thereto over a period of 1 hour. The liquid temperature of both the aluminum sulfate aqueous solution and the fumaric acid aqueous solution was set to 80°C.

After the addition has ended, the reaction solution was further stirred with the impeller to generate the MOF. The reaction time was 1 hour. The pH of the reaction solution was 3 at the start of the reaction and 3.6 at the end of the reaction.

The tap density, average particle size, BET specific surface area, and crystallinity degree of the obtained MOF were measured by the above method. The results are shown in Table 2 below.

[Table 1]

| | Experiment No. | Residence Time | Production Rate | Crystal density | Apparatus volume | Production Rate | Nucleation rate | Linear growth rate | Crystal suspension density | Area-shape factor | Volume-shape factor | Slope of Rosin-Rammler diagram | Particle size characteristic value | Mode diameter based on mass | Integral term | |
|---|---|---|---|---|---|---|---|---|---|---|---|---|---|---|---|---|
| Sign | | | P | pc | V' | P/pcV' | Fv' | dl/dθ | (1-ε) | ka | kv | m | lp | Ld | I1 | I2 |
| Unit | | [min] | [kg/h] | [kg/m³] | [m³] | [/h] | [nuclei/ (hr-m³)] | [mm/h] | [-] | [-] | [-] | [-] | [mm] | [mm] | [-] | [-] |
| Ex. 1 | Run1 | 10 | 1.8 | 1200 | 0.0033 | 0.455 | $2.030 \times 10^{14}$ | 0.0092 | 0.201 | 3.14 | 0.52 | 1.1 | 0.01 | 0.0256 | 0.4075 | 4.3060 |
| | Run2 | 15 | 1.2 | 1200 | 0.0033 | 0.303 | $3.433 \times 10^{13}$ | 0.0097 | 0.209 | 3.14 | 0.52 | 1.05 | 0.015 | 0.0414 | 0.3707 | 5.0291 |
| | Run3 | 30 | 0.6 | 1200 | 0.0033 | 0.152 | $6.767 \times 10^{12}$ | 0.0067 | 0.210 | 3.14 | 0.52 | 1.03 | 0.02 | 0.0570 | 0.3558 | 5.3822 |
| | Run4 | 10 | 5.4 | 1200 | 0.01 | 0.450 | $2.010 \times 10^{14}$ | 0.0087 | 0.210 | 3.14 | 0.52 | 1.1 | 0.01 | 0.0256 | 0.4075 | 4.3060 |
| | Run5 | 15 | 3.6 | 1200 | 0.01 | 0.300 | $3.739 \times 10^{13}$ | 0.0082 | 0.230 | 3.14 | 0.52 | 1.08 | 0.015 | 0.0396 | 0.3929 | 4.5712 |
| | Run6 | 20 | 2.7 | 1200 | 0.01 | 0.225 | $1.475 \times 10^{13}$ | 0.0072 | 0.250 | 3.14 | 0.52 | 1.05 | 0.018 | 0.0497 | 0.3707 | 5.0291 |
| | Run7 | 30 | 1.8 | 1200 | 0.01 | 0.150 | $6.237 \times 10^{12}$ | 0.0056 | 0.260 | 3.14 | 0.52 | 1.01 | 0.02 | 0.0590 | 0.3408 | 5.7810 |
| | Run8 | 15 | 3.2 | 1200 | 0.01 | 0.267 | $4.088 \times 10^{13}$ | 0.0068 | 0.230 | 3.14 | 0.52 | 1.08 | 0.014 | 0.0369 | 0.3929 | 4.5712 |
| | Run9 | 20 | 2.4 | 1200 | 0.01 | 0.200 | $1.557 \times 10^{13}$ | 0.0061 | 0.250 | 3.14 | 0.52 | 1.05 | 0.017 | 0.0469 | 0.3707 | 5.0291 |

[Table 2]

| | Reaction method | Metal | Organic ligand | Nucleation rate [nuclei/(h-m$^3$)] | Linear growth rate [$\mu$m/h] | Residence Time [min] | Tap density [g/cm$^3$] | Average particle size [$\mu$m] | BET specific surface area [m$^2$/g] | Crystallinity degree [%] |
|---|---|---|---|---|---|---|---|---|---|---|
| Ex. 1 | Continuous | Al | Fumaric acid | $6.221 \times 10^{13}$ | 7.6 | 10~30 | 0.6 | 42.4 | 986 | 140 |
| Ex. 2 | Continuous | Al | Terephthalic acid | $8.520 \times 10^{13}$ | 8.2 | 10~15 | 0.55 | 45.1 | 992 | 135 |
| Ex. 3 | Continuous | Zn | Fumaric acid | $8.160 \times 10^{13}$ | 8.8 | 10~15 | 0.51 | 55.2 | 998 | 120 |
| Ref. Ex. 1 | Batch | Al | Fumaric acid | $5.973 \times 10^{14}$ | 2.5 | 60 | 0.33 | 10.2 | 860 | 98 |

18

As is clear from the results shown in Tables 1 and 2, the MOF obtained in each of the examples had a high tap density, large average particle size, and high BET specific surface area and crystallinity degree. In Reference Example 1, where a batch method was employed, an MOF having a high tap density was also obtained. However, the residence time of the reaction solution was long, and the synthesis of the MOF required a long time.

[Example 4]

An experiment was carried out in which the MOF obtained in Example 1 was granulated to create a gas adsorption material. The MOF formed from aluminum fumarate and a thermosetting phenolic resin (Belpar S890, manufactured by Air Water Performance Chemical Inc.) were mixed in powder form to obtain a mixture. The addition ratio of the thermosetting phenolic resin in the mixture was as shown in Table 3.

Next, the mixture was placed in a tumbling granulator having a diameter of 70 cm and mixed. A small amount of water was added to this mixture using a mist sprayer to gradually granulate it.

The granulated materials were sieved to take out granulated material particles that had reached a predetermined particle size, and granulated material particles having particle sizes smaller than the predetermined particle size were returned to the mixture.

The granulated materials that had reached a diameter of 3 mm were heated at the first temperature to remove water and then further heated to the second temperature to cure the thermosetting phenolic resin. The crushing strength and rotational strength of the obtained spherical granulated materials were measured in accordance with JIS Z 8841:1993 and JIS Z 8841:1993. The results are shown in Table 3.

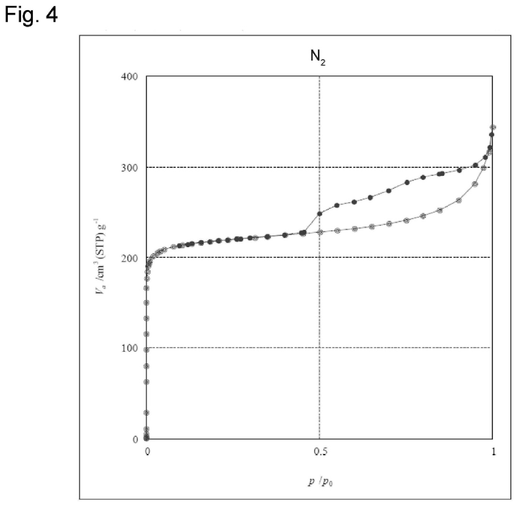

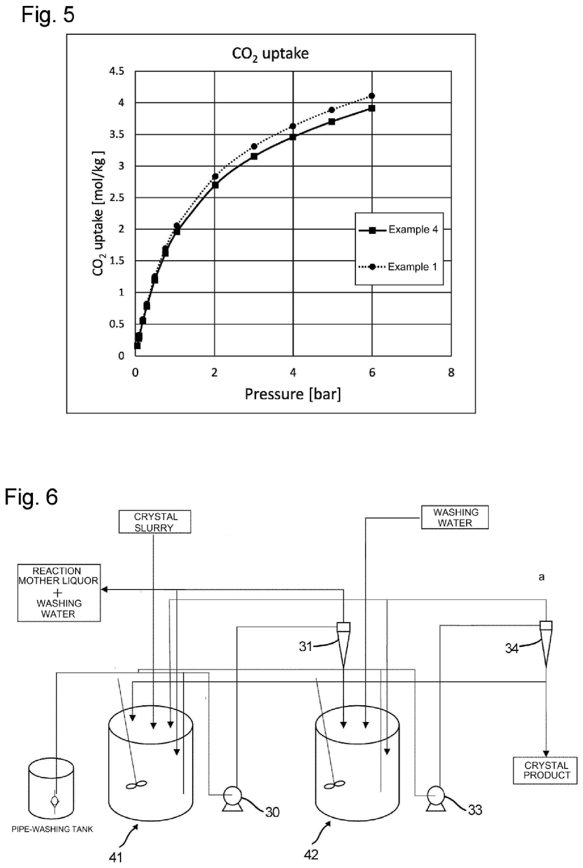

Further, the nitrogen gas adsorption/desorption amount at 77 K and the carbon dioxide gas adsorption amount at 20°C of the obtained spherical granulated materials were measured based on a gas adsorption method. The nitrogen gas adsorption/desorption amount was measured using BELSORP MAX manufactured by Bell Japan, Inc. The carbon dioxide gas adsorption amount at 20°C was measured using iSORB manufactured by Anton Paar GmbH. These results are shown in Figs. 4 and 5. For comparison, Fig. 5 also shows the carbon dioxide gas adsorption amount of the MOF produced in Example 1.

As shown in Fig. 4, the nitrogen gas adsorption/desorption curve of the spherical granulated materials of Example 4 shows a typical Type IV curve according to the IUPAC classification.

Fig. 5 shows the $CO_2$ adsorption amount at 20°C of the MOF of Example 1 and the spherical granulated materials of Example 4. The MOF and spherical granulated materials exhibit high adsorption amounts from 0 bar to 2 bar, and also exhibit high adsorption amounts in a pressurized range of 2 bar or more. Such isothermal adsorption characteristics make them particularly suitable materials for PSA (pressure swing adsorption separation apparatuses) and also suitable for TSA (temperature swing adsorption separation apparatuses). In addition, because the adsorption amount increases even above 6 bar, the material also has potential as a $CO_2$ occlusion material.

Further, as shown in Fig. 5, the $CO_2$ adsorption amount of the spherical granulated materials of Example 4 was approximately 5% lower than the $CO_2$ adsorption amount of the MOF of Example 1, while this reduction was almost the same as the added thermosetting phenolic resin used in producing the spherical granulated materials of Example 4.

[Example 5]

In Example 4, except that the addition ratio of the thermosetting phenolic resin was as shown in Table 3, spherical granulated materials were obtained in the same manner as in Example 4. The obtained spherical granulated materials were measured in the same manner as in Example 4. The results are shown in Table 3.

[Examples 6 and 7]

In Example 4, the addition ratio of the thermosetting phenolic resin was as shown in Table 3. The granulated particle size was also as shown in Table 3. Except for the above, spherical granulated materials were obtained in the same manner as in Example 4. The obtained spherical granulated materials were measured in the same manner as in Example 4. The results are shown in Table 3.

[Comparative Example 2]

A granulated body was produced by an extrusion method. Specifically, a small amount of kaolin and carbon powder as a lubricant were added to an MOF formed from aluminum fumarate (tap density: 0.33 g/cm$^3$, average particle size: 10.2 $\mu$m), and the resulting material was extruded through an opening having a diameter of approximately 3 mm, and was cut to a length of 3 to 4 mm to produce a granulated body. The obtained granulated body was measured in the same manner as in Example 4. The results are shown in Table 3.

[Table 3]

| | Granulation method | Phenolic resin addition ratio [%] | Granulated particle size [mm] | Specific surface area (25°C) [m²/g] | First temperature | | Second temperature | | Crushing strength [N] | Rotational strength [%] |
|---|---|---|---|---|---|---|---|---|---|---|
| | | | | | [°C] | [h] | [°C] | [h] | | |
| Ex. 4 | Tumbling granulation | 5 | 3 | 564.2 | 120°C | 2h | - | - | 35.86 | 0.52 |
| Ex. 5 | Tumbling granulation | 8 | 3 | 550.4 | 120°C | 2h | - | - | 21.33 | 0.61 |
| Ex. 6 | Tumbling granulation | 10 | 5 | 497.2 | 120°C | 0.5h | 170°C | 2h | 33.74 | 0.61 |
| Ex. 7 | Tumbling granulation | 15 | 5 | 481.2 | 120°C | 0.5h | 170°C | 2h | 46.15 | 0.45 |
| Comp. Ex. 2 | Extrusion | 15 | 5 | 353.2 | 120°C | 0.5h | 170°C | 2h | 10.9 | 5.1 |

As is clear from the results shown in Table 3, the granulated body obtained in each of the examples had a strength sufficient for practical use.

[Example 8]

Counterions, which are, here, sodium sulfate, of MOF crystals withdrawn from the reaction vessel 13 shown in Fig. 2 were washed with water using a continuous washing apparatus shown in Fig. 6.

In a first washing step 41, a slurry containing the MOF crystals withdrawn from the reaction vessel 13 and the washing water fed from the second washing step were placed and mixed by stirring. Next, the slurry in the first washing step was transferred to a liquid cyclone 31 by a pump 30, and the slurry containing crystals with large particle sizes and a mother liquor was transferred to a second washing step 42. In the second washing step 42, washing water (pure water) was placed and mixed by stirring. The slurry was transferred to a liquid cyclone 34 by a pump 33, and crystals with large particle sizes and the mother liquor were taken out as a product. Meanwhile, fine particles and the mother liquor were recycled to the second washing step 42.

The obtained MOF crystals were placed in pure water, and the impurity amount was measured by measuring the electrical conductivity of the liquid. An electrical conductivity of 20 mS/m or less was used as the criterion. As a result, the required amount of washing liquid was 500 kg, and the same washing effect was achieved with 50% of the amount of water required in the case of washing by filtration or elutriation.

[Comparative Example 3]

The counterions, which are, here, sodium sulfate, of the MOF crystals withdrawn from the reaction vessel 13 shown in Fig. 2 were washed with water by elutriation.

200 kg of pure water was added to 25 kg of slurry containing the MOF crystals, and the resulting liquid was mixed by stirring and thereafter allowed to stand. Then, the crystals settled after about 2 hours and separated into a crystal layer and a clear mother liquor. Only the clear mother liquor was removed by a pump. 200 kg of pure water was added again, and the resulting liquid was mixed by stirring. The impurity amount of the crystals was obtained by measuring the electrical conductivity. The operation was repeated until the impurity amount reached a predetermined value, specifically, until the electrical conductivity was 20 mS/m or less.

The operation was repeated five times for the electrical conductivity to reach 20 mS/m or less. Therefore, the total amount of pure water used for washing was 1,000 kg.

Industrial Applicability

According to the present invention, an MOF having high crystallinity and high bulk density can be easily produced.

**Claims**

1. A metal-organic framework comprising:
a crystal containing an organic ligand and a metal ion,

    wherein the organic ligand is a fumaric acid dianion or a terephthalic acid dianion,
    the metal ion is an aluminum ion, a zinc ion, or an iron(III) ion, and
    the metal-organic framework has a tap density of 0.3 g/mL or more and 0.9 g/mL or less.

2. The metal-organic framework according to claim 1, wherein the crystal is an aluminum fumarate crystal.

3. A gas adsorption material comprising a granulated body of a mixture including: the metal-organic framework according to claim 1 or 2 as a gas adsorption component; and a phenolic resin as a shape-retaining component.

4. The gas adsorption material according to claim 3, having a crushing strength of 20 N or more, and a rotation strength of 3% or less, the rotation strength being evaluated using a rolling device.

5. A production method for producing a metal-organic framework using a crystallizer including a reagent introduction zone, a crystallization zone, a circulation zone, and a recovery zone,
the method comprising:

    in the reagent introduction zone, simultaneously and continuously adding a first aqueous solution containing a metal ion and a second aqueous solution containing an organic ligand capable of coordinating to the metal ion through different nozzles installed in a flow path, and immediately mixing the first aqueous solution and the second aqueous solution using a mixing mechanism;
    in the crystallization zone, reacting the metal ion and the organic ligand to grow a crystal of a metal-organic framework and simultaneously nucleate the crystal of the metal-organic framework;
    in the circulation zone, circulating a slurry containing the crystal of the metal-organic framework to a reagent introduction step; and
    in the recovery zone, withdrawing a portion of the slurry continuously or at a predetermined interval.

6. The production method according to claim 5, wherein the first aqueous solution and the second aqueous solution are added such that a nucleation rate of the crystal is $1\times10^5$ nuclei/(h·m$^3$) or more and $1\times10^{16}$ nuclei/(h·m$^3$) or less.

7. The production method according to claim 5 or 6,
wherein the first aqueous solution and the second aqueous solution are added such that a linear growth rate of the crystal is 1 μm/h or more and 200 μm/h or less.

8. The production method according to claim 5 or 6,
wherein the crystal is withdrawn from the crystallizer such that a residence time in the crystallizer including all of the reagent introduction zone, the crystallization zone, the circulation zone, and the recovery zone is 3 minutes or more and 30 minutes or less.

9. The production method according to claim 5 or 6, wherein the crystallizer is of a draft tube baffle type.

10. The production method according to claim 9,
wherein in the crystallizer of the draft tube baffle type, a sum of a volume of the reagent introduction zone and a volume of the crystallization zone is 30% or less of a volume of the crystallizer.

11. The production method according to claim 5 or 6,

    wherein the metal ion is an aluminum ion,
    the organic ligand is a fumaric acid dianion, and
    0.9 mol or more and 1.1 mol or less of the fumaric acid dianion is added per mol of the aluminum ion.

12. The production method according to claim 5 or 6,

    wherein the metal ion is an aluminum ion,

the organic ligand is a terephthalic acid dianion, and
0.9 mol or more and 1.1 mol or less of the terephthalic acid dianion is added per mol of the aluminum ion.

13. The production method according to claim 5 or 6,

wherein the metal ion is an iron(III) ion,
the organic ligand is a terephthalic acid dianion, and
0.9 mol or more and 1.1 mol or less of the terephthalic acid dianion is added per mol of the iron(III) ion.

14. The production method according to claim 5 or 6,

wherein the metal ion is a zinc(II) ion,
the organic ligand is a fumaric acid dianion, and
0.9 mol or more and 1.1 mol or less of the fumaric acid dianion is added per 4 mol of the zinc(II) ion.

15. A continuous washing method for continuously washing the metal-organic framework produced by the method according to claim 5,

the continuous washing method comprising:
a washing step of washing the metal-organic framework with a washing liquid to wash and remove a counteranion for dissolving the metal ion constituting the metal-organic framework and a countercation for dissolving the organic ligand,
wherein the washing step includes a first washing step, a solid-liquid separation step, and a second washing step, and
a liquid that has been subjected to the first washing step is separated into a solid component and a liquid component in the solid-liquid separation step, the solid component is further washed in the second washing step, and the liquid component is used as a part of a washing liquid in the first washing step.

16. The continuous washing method according to claim 15, wherein in the solid-liquid separation step, solid-liquid separation is carried out using a centrifugal force.

17. A production method for producing a gas adsorption material, comprising:

a step of mixing the metal-organic framework produced by the method according to claim 5 with a thermosetting phenolic resin to obtain a mixture;
a step of adding water to the mixture and performing tumbling granulation to obtain granulated materials;
a step of separating the granulated materials that have grown to a predetermined particle size, in accordance with particle sizes thereof; and
a step of again subjecting the granulated materials that do not reach the predetermined particle size to the granulating step, heating the granulated materials that have reached the predetermined particle size at a first temperature to remove the water, and further heating the granulated materials that have reached the predetermined particle size to a second temperature to cure the thermosetting phenolic resin.

## Fig. 1

Fig. 2

Fig. 3

(a)

(b)

Fig. 4

## Fig. 5

CO$_2$ uptake

Legend: Example 4, Example 1

Y-axis: CO$_2$ uptake [mol/kg]
X-axis: Pressure [bar]

## Fig. 6

## INTERNATIONAL SEARCH REPORT

| International application No. |
| --- |
| **PCT/JP2024/033770** |

**A. CLASSIFICATION OF SUBJECT MATTER**

*C07C 57/15*(2006.01)i; *B01J 20/22*(2006.01)i; *B01J 20/30*(2006.01)i; *C07C 63/16*(2006.01)i; *C08K 5/56*(2006.01)i; *C08L 101/00*(2006.01)i; *C07F 3/06*(2006.01)n; *C07F 5/06*(2006.01)n
FI: C07C57/15; B01J20/22 A; B01J20/30; C07C63/16; C08L101/00; C08K5/56; C07F3/06; C07F5/06

According to International Patent Classification (IPC) or to both national classification and IPC

**B. FIELDS SEARCHED**

Minimum documentation searched (classification system followed by classification symbols)

C07C57/15; B01J20/22; B01J20/30; C07C63/16; C08K5/56; C08L101/00; C07F3/06; C07F5/06

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
| --- | --- | --- |
| X | JP 2018-538284 A (BASF SOCIETAS EUROPAEA) 27 December 2018 (2018-12-27) examples 12, 13 | 1, 2 |
| Y | | 3-16 |
| A | | 17 |
| X | JP 5960144 B2 (BASF SOCIETAS EUROPAEA) 02 August 2016 (2016-08-02) examples 5, 6 | 1, 2 |
| Y | | 3-16 |
| A | | 17 |
| X | JP 2021-533972 A (COMMONWEALTH SCIENTIFIC AND INDUSTRIAL RESEARCH ORGANISATION) 09 December 2021 (2021-12-09) paragraphs [0176]-[0185] | 1, 2 |
| Y | | 5-16 |
| A | | 17 |

[✓] Further documents are listed in the continuation of Box C.    [✓] See patent family annex.

| | |
| --- | --- |
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| **14 November 2024** | **03 December 2024** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

**INTERNATIONAL SEARCH REPORT**

International application No.

**PCT/JP2024/033770**

C. DOCUMENTS CONSIDERED TO BE RELEVANT

| Category* | Citation of document, with indication, where appropriate, of the relevant passages | Relevant to claim No. |
|---|---|---|
| Y | JP 2022-050208 A (NIX, INC.) 30 March 2022 (2022-03-30) examples 2, 13, 24, 35, 46, 57, 68, 79, 90, 101, 111, 122, 134 | 3, 4 |
| A | | 1, 2, 5-17 |
| Y | JP 2019-166499 A (SUMITOMO METAL MINING CO., LTD.) 03 October 2019 (2019-10-03) fig. 1 | 5-16 |

Form PCT/ISA/210 (second sheet) (July 2022)

## INTERNATIONAL SEARCH REPORT

International application No.

**PCT/JP2024/033770**

| Box No. III | Observations where unity of invention is lacking (Continuation of item 3 of first sheet) |
|---|---|

This International Searching Authority found multiple inventions in this international application, as follows:

In claims 1-17, there are a plurality of independent claims, and the unity of invention should be considered primarily in relation to the independent claims other than dependent claims (Annex B(c) of Administrative Instructions under the Patent Cooperation Treaty).

When considering the relationship between claim 1 and claim 5, which are independent claims, the technical feature common to these independent claims is a "metal-organic framework." However, this feature does not have novelty in light of JP 2018-538284 A, JP 5960144 B2, and the like, and thus is not a special technical feature. There are no other same or corresponding special technical features between these independent claims.

Therefore, there is no unity between claims 1-17.

Therefore, claims are classified into two inventions below.
(Invention 1) Claims 1-4
(Invention 2) Claims 5-17

1. ☐ As all required additional search fees were timely paid by the applicant, this international search report covers all searchable claims.

2. ☑ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**   ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (July 2022)

## INTERNATIONAL SEARCH REPORT
### Information on patent family members

International application No.

**PCT/JP2024/033770**

| Patent document cited in search report | | | Publication date (day/month/year) | Patent family member(s) | | | Publication date (day/month/year) |
|---|---|---|---|---|---|---|---|
| JP | 2018-538284 | A | 27 December 2018 | US 2018/0333696 example 12, 13 | A1 | | |
| | | | | WO 2017/089410 | A1 | | |
| | | | | CN 108290134 | A | | |
| | | | | KR 10-2018-0088679 | A | | |
| JP | 5960144 | B2 | 02 August 2016 | WO 2012/042410 examples 5, 6 | A1 | | |
| | | | | CA 2812032 | A1 | | |
| | | | | CN 103140495 | A | | |
| | | | | KR 10-2013-0113462 | A | | |
| JP | 2021-533972 | A | 09 December 2021 | US 2020/0282379 paragraphs [0215]-[0222] | A1 | | |
| | | | | US 2020/0363078 | A1 | | |
| | | | | US 2022/0176344 | A1 | | |
| | | | | WO 2020/034008 | A1 | | |
| | | | | CN 111565817 | A | | |
| | | | | CA 3084289 | A1 | | |
| | | | | KR 10-2021-0042043 | A | | |
| JP | 2022-050208 | A | 30 March 2022 | (Family: none) | | | |
| JP | 2019-166499 | A | 03 October 2019 | (Family: none) | | | |

Form PCT/ISA/210 (patent family annex) (July 2022)

## REFERENCES CITED IN THE DESCRIPTION

**Patent documents cited in the description**

- US 20220220129 A1

**Non-patent literature cited in the description**

- Basics of Materials Science. *Basics of Porous Coordination Polymers (PCP)/Metal-Organic Frameworks (MOF)*, September 2012, vol. 7, https://www.sigmaaldrich.com/JP/ja/campaigns/materials-science-basic>

- *CHEMICAL ABSTRACTS*, 1370461-06-5